# EUROPEAN PATENT APPLICATION

(11) **EP 2 002 732 A1**
(43) Date of publication of application: **17.12.2008**
(21) Application number: 07109854.5
(22) Date of filing: 08.06.2007
(51) Int. Cl.: A23L 1/052, A23L 1/212, A23L 1/0534

(54) **Fruit fiber gel**

(71) Applicant: Nestec S.A., 1800 Vevey (CH)
(72) Inventor: Gehin-Delval, Cécile, 25160 LABERGEMENT SAINTE MARIE (FR); Redgwell, Robert John, 1073 SAVIGNY (CH); Curti, Delphine Gisèle, 1042 BETTENS (CH); Folmer, Britta, 1005 LAUSANNE (CH); Labat, Emilie, 1007 LAUSANNE (CH); Raemy, Alois, 1814 LA TOUR-DE-PEILZ (CH)
(74) Representative: Marquardt, Ulf

(57) **Abstract**

Gelification is an important topic in many types of consumer products today, such as foods, cosmetical or medical products but also in paints or adhesives. The present invention relates to the field of gels and gelling material and their applications.

In particular, the present invention relates to a gel comprising a liquid medium and a gel matrix comprising sheared cell wall material from ripe and soft botanical fruits, its preparation, its possible uses, a product comprising such a gel and the corresponding gel building material and its application.

## Description

The present invention relates to the field of gels and gelling material and their applications.

Gelification is an important topic in many types of consumer products today, such as foods, cosmetical or medical products but also in paints or adhesives.

For centuries chefs have used gelification to turn liquid fruit juice into set jam or to turn liquid broth into thicker sauces. These techniques add elements of convenience, improved mouthfeel or simply a new texture to improve the experience of food.

Gelification is used to produce creamier products, to generate a more indulgent mouthfeel and/or to improve freeze/thaw stability and increase water retention.

In the food industry gelification is usually achieved today by the application of gelling agents such as starch, gelatine, agar, β-glucan, guar gum, carragenan, or alginate. However, all these gelling agents have in common that they have a relatively high caloric content or carry an E-number (E-number is a code for a chemical additive, which should be avoided in the food insustry) and additionally, at least some of them are relatively expensive.

Furthermore, food products often require heating or cooling. It would be desirable to have available a gel or a gelling material to produce a gel that is stable in broad temperature ranges as well as when it is subjected to variations in temperature.

These problems are well recognized in the art.

Attempts have been made to overcome these problems by using fibres, obtained from cereal for fat: they are used as a partial fat replacement in cheese (Patent N° US 2006/0034996 A1) or in sauces (Patent n° US 2006/0034998 A1). Similarly, in the patent n° US2001/0001677 A1, the inventors used a wheat fibre gel and starch as a fat replacement in yoghurts.

However, gels generated by fibres obtained from cereal have the disadvantage that a labour extensive treatment of the cereal is required that is both, costly and time consuming, to arrive at the fibres that can be used for gelification. Further, their water capacity is about 24 times of the weight of the fibres, which is relatively low for a gel.

In sports industry today it is desirable to deliver a feeling of freshness and hydration during endurance sports to the athlete, however this is problematic as ingesting water gives an uncomfortable feeling in the stomach: too much liquid sloshing in the stomach.

Existing sport products are either beverages that have a high sugar content, or gels that have a low water content. Both leave a thirsty feeling in the mouth.

It would hence be desirable to have available a gel that provides a nutritional product for athletes that quenches the thirst without conferring an uncomfortable feeling in the stomach.

The above mentioned disadvantages of the prior art were addressed and overcome by the present inventors.

It was the object of the present invention to provide the art with a gel or a product comprising a gel that is stable in an extended temperature range, has a neutral taste and a pleasant texture, that can be used as gel, as binder or to stabilize foams and that comprises a gelling material that has a low caloric content, a high capacity for liquids, is inexpensive and is obtainable from natural sources with a simple and fast process.

This object is solved by a gel in accordance with claim 1, a use in accordance with claim 16 or 21, a product in accordance with claim 17, a gel building material in accordance with claim 19 and a method in accordance with claim 22.

In addition the cell wall based gels have a less slimy and sandy or gritty feeling and possess a more natural pulpiness in the mouth

The present inventors were surprised to find that a gel comprising a liquid medium and a gel matrix comprising sheared cell wall material from ripe and soft botanical fruits achieves the object of the present invention.

In a preferred embodiment a gel comprising a liquid medium and a gel matrix comprising sheared cell wall material from ripe and soft botanical fruits that was dried and/or washed with organic solvents achieves the object of the present invention.

Using this gel matrix it is possible to prepare a gel made of, e.g, 1 weight-% of extracted fruit cell wall material in 99 weight-% of water. This gel was found to be stable over more than 12 months at 4°C without syneresis.

Consequently, one embodiment of the present invention is a gel comprising a liquid medium and a gel matrix comprising sheared cell wall material from ripe and soft botanical fruits.

For the purpose of the present invention the gel is a colloid in which the disperse phase has combined with the dispersion medium to produce a semisolid material, e.g., a jelly. A system is gel-like at a given frequency as soon as G' (storage modulus which concerns the solid part of the material) is higher than G" (loss modulus which concerns the liquid-like response on the material).

A gel matrix is for the purpose of the present invention a material that is capable of forming a porous network of interacting particles that spans the volume of a liquid medium in a gel. The form of interactions by which the particles are interconnected is not critical for the gel matrix, any form of interaction is possible. It is preferred, however, that the particles interact at least in part by electrostatic interactions.

Shearing means that the native structure of a material, such as cell wall material, is at least partially disrupted. This disruption can be achieved, e.g., by chemical or by physical means. Chemical means could be, e.g., a treatment at a high pH (e.g., 10-12) or at a low pH (0-3). Preferably, however, shearing is achieved by subjecting the material to physical stress. Physical stress can be applied, e.g., by a blender or by grinding. In one embodiment of the present invention it is preferred that the stress is applied by a high shear treatment with a Ultra-Turrax operating at about 10000rpm to 40000rpm, preferably about 24000 rpm for about 10s-60s, preferably about 30 s.

Cell wall material is generally any material that comprises cell walls. Preferably, cell wall material is a material that is usually discarded during the production of products, such as, e.g., fruit juices. Preferably cell wall material comprises the core tissue and/or the pericarp and locule tissue of fruits. This cell wall material is preferably further treated to remove liquid components, e.g., by centrifugation and optionally subjected to washing steps. Preferably the cell wall material has a moisture content of below 10 %, more preferred of below 1 %, even more preferred of below 0.1 %.

Fruits are considered ripe for the purpose of the present invention after a burst of ethylene production - an important plant hormone involved in ripening - took place. Often times, ripe fruits can be easily discriminated from unripe fruits by the occurrence of a change in color, in texture and or in taste. For example, in a preferred embodiment of the present invention a fruit is to be considered ripe if its sugar content compared to its level before the ethylene burst is increased by at least 20 %.

Fruits are considered soft if their firmness by a pressure measurement is below 15 N/g, preferably below 10 N/g, most preferred about 2-6 N/g.

The present invention makes reference to botanical fruits. Botanical fruits are fruits in accordance with the botanical definition of fruit and include both, culinary fruits, such as, e.g., peaches, kiwi, plums, etc. and vegetables, such as, e.g., tomatoes, avocados, etc.

The liquid medium is not particularly limited for the purpose of the present invention and can be a polar liquid or an nonpolar liquid or a mixture thereof.

The unpolar liquid can comprise, e.g., one or more non-volatile oils extracted from plants, usually the seeds; one or more essential oils, such as volatile aromatic oils extracted from plants; one or more animal fats; or mixtures thereof.

The polar liquid can comprise, e.g., water based liquids, such as fruit juice, coffee or tea.

The liquid can also be an emulsion, e.g., milk, milk-like products, e.g., soy milk, or milk based products such as chocolate drinks or malt drinks.

In a preferred embodiment of the present invention the cell wall material is obtained from the parenchymatous tissue. Parenchyma cells are thin-walled cells of the ground tissue that make up the bulk of most nonwoody structures. They can have a variety of functions and are relatively abundant in tissues. The nature of parenchymatous tissue allows it to obtain cell wall material in high yields that - if sheared - has a very high liquid capacity in a gel.

The cell wall material in accordance with the present invention can be chemically modified to confer certain additional properties to the cell wall material. Possible modifications would be the addition of coloured material, the introduction of sugar moieties or the insertion of further cross linking agents to improve gel stability. The introduction of such functionalities is well within the skill of those skilled in the art.

However, in particular for food applications it is preferred that the cell wall material as a natural product is chemically unmodified to comply with the growing consumers desire today to prefer natural products.

It was found that a particularly effective gel building material is obtained when the cell wall material is sheared, preferably to an average fibre length of about 100 - 0.01 µm, preferably of about 10 - 0. 1 µm, most preferred of about 2 - 0.1 µm.

To produce a gel the sheared cell wall material from soft botanical fruits can well be combined with other gel building materials that are known in the art, e.g., starch, gelatine, agar, β-glucan, guar gum, carragenan, alginate, and/or fibers from cereal.

In a preferred embodiment the gel matrix of the present invention comprises at least 10 weight-%, preferably at least 30 weight-%, more preferred at least 50 weight-%, even more preferred at least 75 weight-% and still more preferred at least 90 weight-% cell wall material from soft botanical fruits.

In a particular preferred embodiment of the present invention the gel is characterized in that the gel matrix consists of cell wall material from soft botanical fruits.

The sheared cell wall material in accordance with the present invention can be obtained by any method that is known in the art.

However, in a preferred embodiment of the present invention the sheared cell wall material is obtainable by a method comprising the following steps:
- crushing the fruits,
- optionally stopping the enzymatic activity within the fruit material, preferably by a heating step
- separating the cell wall material from the remaining constituents, preferably by the use of a separator or by centrifugation,
- optionally washing to further purify the cell wall material.
- shearing
- optionally drying in organic solvents

Instead of crushing the fruits it is also possible to use the waste material, e.g., from botanical fruit juice, marmalade or Ketchup production.

Optionally, further agents might be added such as ascorbic acid to prevent browning.

Crushing the fruits will have the consequence that several enzymes are freed and can be active on the cell wall material. In order to avoid that this enzymatic activity impairs the quality of the cell wall material, a heating step can be carried out to inactivate the enzymatic activity. Of course, other known methods to inactivate enzymatic activity can be used equally well. The heating step is preferably carried out by heating to 60-120 °C, preferably to about 80 °C for 5-30 minutes, preferably for 10 minutes.

The separation of the cell wall material from the remaining constituents of the fruits can be carried out by any means that is known to those skilled in the art. For example, this can be accomplished by using a separator or a centrifuge and by following the manufacturer's instructions.

For example the tissue suspension can be centrifuged at 2000-7000 g preferably at about 5000 g for 2 min-1 hour, preferably for about 10 min. The supernatant is discarded.

Optionally further one or more washing steps can be applied. For example, the residue can be re-suspended in water and centrifuged again to remove the supernatant. Washing steps have the advantage that the cell wall material that is obtained has a high degree of purity which will contribute to the homogeneity of the resulting gel.

In a particular preferred embodiment of the present invention the resulting cell wall material is subjected to at least one washing and/or drying step in at least one organic solvent. The organic solvent can be
for example a polar solvent, e.g., selected from the group consisting of acetone, ethyl alcohol, propanol, isopropanol, and non-polar solvents e.g.hexane, benzene, toluene, diethyl ether, chloroform, ethyl acetate, dichloromethane; or
for example a polar aprotic solvent, e.g., selected from the group consisting of 1,4-dioxane, tetrahydrofuran (THF), acetonitrile (MeCN), dimethylformamide (DMF), dimethyl sulfoxide (DMSO); or
mixtures thereof.
Acetone is in particular preferred.
For example this washing and drying step in an organic solvent can be carried out as follows: The residue is resuspended in acetone and centrifuged as before. The acetone supernatant is then discarded and optionally this step can be repeated. Finally, the residue is resuspended in acetone again and filtered through a filter, e.g., a glass fibre paper (GF/A). The remaining acetone is then allowed to evaporate. This process can be supported by means known in the art, for example by application of a vacuum or by the application of heat.

The present inventors have found that surprisingly this drying step in organic solvents allows to increase the water-retaining capacity of the resulting cell wall material. After shear this can increase even more to about 100-200 mL/g cell wall material, for example to about 180 mL/g cell wall material if the cell wall material is obtained from tomatoes.

Shearing can be carried out as described above. For example the cell wall material in suspension can be submitted to high shear treatment or high pressure homogenisation leading to the size reduction of the cell wall material so that fibres with a diameter between 0.1 and 10µm are obtained.

In one embodiment of the present invention the soft botanical fruits are selected from the group consisting of soft vegetables and soft culinary fruits.

Many foods are botanical fruits but are treated as vegetables in cooking. These include, e.g., cucurbits (e.g., squash, pumpkin, and cucumber), tomato, peas, beans, corn, eggplant (aubergine), and sweet pepper, spices, such as allspice and chillies. Occasionally, though rarely, a culinary "fruit" will not be a true fruit in the botanical sense. For example, rhubarb is considered a culinary fruit for the purpose of the present invention, though only the astringent petiole is edible. In the culinary sense, a fruit is any sweet tasting plant product associated with seed(s), whereas a vegetable is any savoury or less sweet plant product.

In a preferred embodiment of the present invention the soft botanical fruits are selected from the group consisting of tomato, kiwi, plums, strawberries, cherries, kaki (persimmon), or mixtures thereof. In particular preferred are tomato and/or kiwi.

In one embodiment of the present invention the soft botanical fruits are fruit that exhibit an in-vitro swelling of the cell walls during the ripening process, preferably a swelling ratio of ripe fruit vs. unripe fruit of ≥ 2.5. In accordance with this, botanical fruit are also considered ripe in the context of the present invention if the cell walls have swelled compared to the unripe fruit at least about 2.5-fold following microscopic examination.

The liquid medium in the gel of the present invention is preferably a polar medium, preferably comprising water.

The liquid medium can also comprise a non-polar medium, possibly as an emulsion. Such an emulsion may comprise water and fats, preferably milk fat, long chain fatty acids, medium chain triglycerides, ω3-fatty acids, ω6 fatty acids, plant oils, fish oils or mixtures thereof, in particular preferred is the emulsion milk, a milk-like or a milk based product. A milk-like product can be soy milk or products based thereon.

In one embodiment of the present invention the gel is foamed. Foaming can take place by the introduction of a gas or of a mixture of gases during the gel building process or briefly prior to the addition of the gel building material to the liquid to be gelled. In principle any substance that is present in gas form at the intended temperature of use of the resulting product is applicable. Which gases can be used depends only on the intended purpose of the product. For food applications air, nitrogen, carbon dioxide or noble gases are preferred. Consequently the gel and the gel building material of the present invention can be used to stabilize foams. This property will be of importance, e.g., when trying to conserve foams on food products for later consumption during storage times.

Optionally the gel of the present invention can further comprise one or more additives selected from the group consisting of sweeteners, in particular sugars, buffers, colouring agents, preservatives, salts, spices, vitamins, flavouring ingredients, carbohydrates, proteins, bioactives, minerals, acidifying agents, medicaments, or mixtures thereof.

An embodiment of the gel of the present invention is in particular characterized in that the liquid medium represents 60-99.9 weight-% of the gel, preferably 90-99.6 weight-% of the gel, in particular 98-99.5 weight- % of the gel and/or the cell wall material represents 10-0.1 weight-% of the gel, preferably 5.0-0.4 weight-% of the gel, in particular preferred 2-0.5 weight-% of the gel.

One embodiment of the present invention is characterized in that the gel, and consequently the gel building material consists of edible material. Edible material comprises preferably food-grade material.

The scope of the present invention comprises a product comprising the gel of the present invention.

Such products can be, e.g., cosmetical or a medical product, in particular a moisturizing gel, a shaving cream, a shower gel, a cream or a gel for topical application or a food product, in particular an ambrosia, a jello, a pudding, an ice cream, a confectionery product, a sauce or a functional sports food such as a hydrating gel. Further possible products are gelified water for everyday life (with aroma and differentiation through packaging), sports gels, gelled water for dried mouth prevention, gelled water with nutrients for elderly, spoonable fruit juice for growing up children, gelling agent in dessert or a jelly layer in a dessert.

In particular in food products the gels of the present invention help to control calorie intake. Notably the gel made from insoluble fruit fibres according to the present invention has a low caloric value itself and offers, hence, a big advantage if it is desired to produce the same mouthfeel and richness as in products, e.g., made with starch based gels, however with a significantly reduced calorie content.

Notably, the gel of the present invention is stable in a broad temperature range that comprises the temperature range that is relevant for food applications from about -25°C to 120°C. Without wanting to be bound by this theory the inventors believe that this effect is observed because the interactions within the gel that provide for the gel stability are mainly electrostatic interactions, which are strong enough to endure temperature variations.

A further remarkable advantage of the gel and of the gel building material of the present invention is that no heating is required to produce a gel. This makes the preparation of the gel safe for children and saves energy.

The gel of the present invention is also storage stable for several months, in particular for about 1-6 months prior to consumption.

The gel of the present invention furthermore is stable at high salt conditions. For Example at a salt concentration of up to 1 M salt, a concentration of cell wall material of at least 2.5% is sufficient to generate a gel. Preferred salt concentrations of 10 mM to 500 mM can be used.

All these properties make the gel of the present invention in particular applicable to food products.

The remarkable ability of the sheared cell wall material of the present invention that comprises insoluble fibres extracted from fruit to be able to form a gel containing about 99 weight-% water makes the gel of the present invention particularly applicable as hydrating gel.

Such a product could be, e.g., used in the cosmetic field or could be taken by athletes for hydration without causing stomach discomfort.

In addition, the colloidal properties of the gel make it possible to entrap beneficial minerals and nutrients in the gel, and to control their release and/or to mask their taste.

Furthermore, the gel and the gel building material of the present invention helps to prevent or relieve constipation. Insoluble fiber adds bulk to the food increasing the rate at which food passes through the digestive tract, thus relieving constipation. It reduces the risk of bowel cancer. When food moves through the digestive tract quickly, there is less time left for harmful substances to build up in the intestine. This may help prevent bowel cancer. Finally it helps to prevent hemorrhoids. Insoluble fiber makes food move faster through the intestine. This reduces pressure in the intestine, the main reason for hemorrhoids.

The present invention also relates to a gel building material that is used to prepare the gel of the present invention and represents the gel matrix in the gel.

Consequently, one embodiment of the present invention is a gel building material comprising sheared cell wall material from ripe, soft botanical fruits. Preferably, the cell wall material is chemically unmodified.

Those skilled in the art will understand that the same considerations made with respect to the gel matrix and/or the cell wall material will equally apply to the gel building material.

In particular, the gel building material can be obtained from ripe soft botanical fruits by any method that is known in the art.

In a preferred embodiment the gel building material of the present invention is obtainable by a method comprising the following steps:
- crushing the fruits,
- optionally stopping the enzymatic activity within the fruit material, preferably by a heating step,
- optionally shearing the cell wall material
- separating the cell wall material from the remaining constituents, preferably by the use of a separator or by centrifugation,
- optionally washing to further purify the cell wall material
- optionally drying in organic solvents.

For this process the same considerations apply as listed above with respect to the sheared cell wall material.

While the gel building material according to the present invention is preferably used to produce a gel it can also be used as a binder. Using gel building material as a binder offers the same advantages as mentioned for the gel. In particular, the caloric content can be reduced and the beneficial health effects from presence of insoluble fibre can be exploited.

The gel of the present invention can be prepared by several methods. If a fruit gel is desired it is possible to use the soft botanical fruit material directly and to shear the cell wall material in this fruit material after adjusting the liquid content appropriately. The result will be a fruit gel.

Notably, the gel strength of the gel can simply be adjusted by either adjusting the amount of liquid or the amount of cell wall material. A further possibility would be the addition of another gel building material that are known in the art and that are exemplified above.

The gel building material can also be prepared initially without the shearing step. This has the advantage that the gel building material can then be added to liquid to be gelled and gellation can be initiated just in time by shearing.

Alternatively the gel building material of the present invention can be added to the liquid to be gelled. This way gellation will start immediately after addition.

Other ways of preparing the gel of the present invention will be apparent to those of skill in the art and are comprised by the subject matter of the present invention.

Those skilled in the art will understand that they can combine any features described in this specification without departing from the scope of the invention as disclosed.

Further embodiments and advantages of the present invention will be evident from the following Examples and Figures.

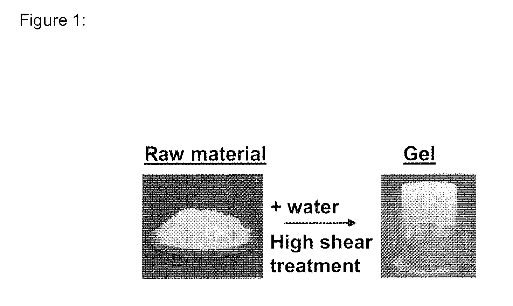

Figure 1 shows the formation of the gel when a 1% suspension of cell wall material of the present invention is subjected to a high shear treatment for 60 seconds.

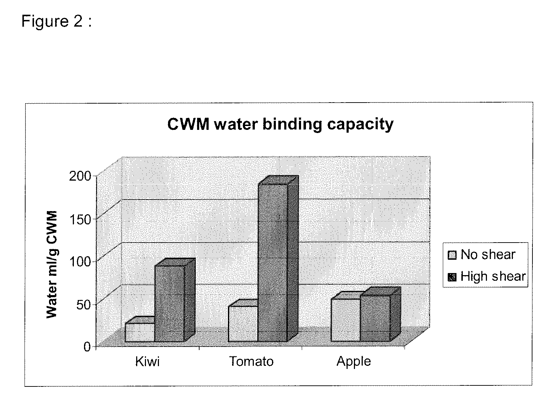

Figure 2 shows the water-retaining capacity of cell wall material prepared from ripe kiwis, tomatoes and apples. The results show that the enhanced water-retaining capacity following high shear is restricted to cell wall material from ripe kiwi and tomato.

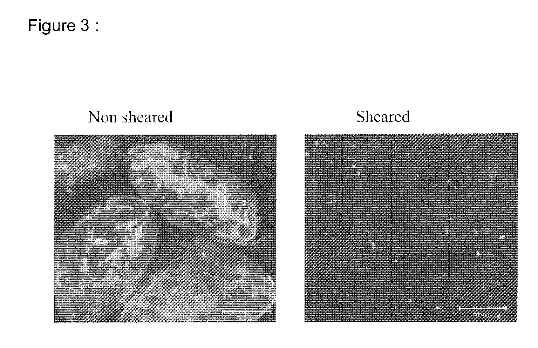

Figure 3 is a confocal micrograph of a 1% suspension of tomato cell wall material before and after high shear treatment.

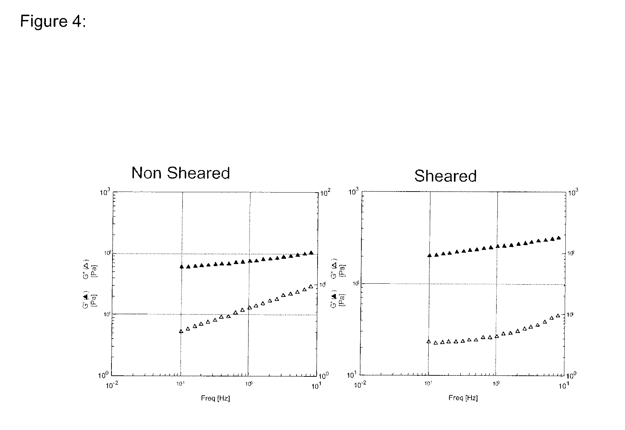

Figure 4 presents the results of a rheological measurement showing an increase in gel forming capacity following a shearing treatment.

Figure 5 compares the viscosifying properties of cell wall material from tomatoes prepared in accordance with the present invention with two commercially available tomato fibres.

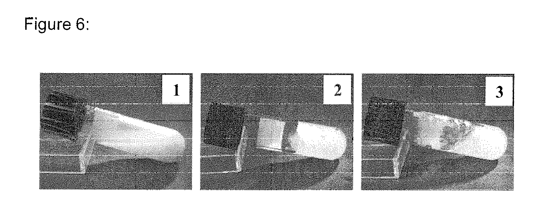

Figure 6 shows examples of gels prepared in accordance with the present invention: Panel 1 shows a PowerBar^{®} Gel with vanilla flavor; panel 2 shows a gel made of 1 wt% gel building material from tomatoes in pure water; panel 3 shows a gel made of 1 wt% gel building material from tomatoes with PowerBar^{®} material (41 g/200 ml).

### Example 1:

### Preparation of tomato cell wall material (CMW) with enhanced water retaining properties

Ripe tomatoes were immersed in water at 100°C for 60 sec. The skin was removed, the locule region and seeds discarded and the pericarp tissue recovered. Pericarp tissue was placed in a kitchen blender with some added water and homogenised. The tissue homogenate was heated to 80° C for 10 min, cooled to room temperature and centrifuged for 10 min at 7268g. Sediment (cell wall material, CWM) was washed by re-suspending in water (3 x) and centrifuging after each addition. It was then re-suspended in acetone (2x) and centrifuged each time to recover the CWM. CWM was resuspended in acetone, filtered through a GF/A glass fibre paper and finally washed with additional acetone. The cake of CWM was broken into particles and allowed to air dry.

CMW from Red Matrix Tomatoes was obtained, e.g., as follows:

6.9 kg Red Matrix tomatoes were soaked for 60 seconds in boiling water. Skin, seeds and coarse were removed thereafter by hand. 4.4 kg fruit flesh were obtained. This flesh was homogenized in a kitchen blender and thereafter heat treated at 80°C for 10 minutes. The homogenized flesh was cooled down to room temperature by the application of cool water and afterwards centrifuged at 5000 rpm (7268 g) for 10 minutes. The supernatant was discarded and the remaining cellular material was washed with 500 ml water. The resulting slurry was centrifuged again at 5000 rpm (7268 g) for 10 minutes. This washing step including the subsequent centrifugation was carried out in total 3 times.

The obtained cellular material was split into two fractions in a ratio of 2:1.

2/3 of the obtained cellular material was washed with 500 ml acetone p.a. followed y a centrifugation step at 5000 rpm (7268 g) for 10 minutes. This washing procedure in acetone as organic solvent was in total repeated three times. The obtained washed cellular material was then passed through a Buchner funnel equipped with a GF/A filter and again washed with more acetone as organic solvent. Afterwards the cellular material was dried under a fume hood to obtain 20.5 g CMW from Red Matrix tomatoes.

The remaining 1/3 of the cellular material obtained after washing with water was subjected to the exact same treatment as described above, however with the difference, that ethanol (94%) was used instead of acetone. 10.7 g CMW from Red Matrix tomatoes were obtained.

### Example 2:

### Preparation of gel from tomato CWM

CWM, (0.5g) prepared as described above, was suspended in 50 ml of water and left at ambient temperature for 30 min. The suspension was then subjected to homogenisation at 24,000 rpm in an Ultra-Turrax for 60 second. Following this treatment a stable gel was formed.

Typical preparation examples are:
Pure hydrating gel
   1 wt% cell wall material
   99 wt% water
Hydrating gel comprising electrolytes
   1 wt% cell wall material
   0.5 wt% Na⁺
   0.05 wt% K⁺
   0.82 wt% Cl⁻
   97.63 wt% water
Hydrating gel comprising nutrients With Power gel composition:

### Ingredients:

Maltodextrin, Filtered Water, Fructose, PowerBar® Electrolyte Blend (Sodium Chloride, Sodium Citrate, Potassium Chloride), Natural Flavor, Citric Acid, I-leucine (170mg/100g), I-valine (170mg/100g), I-isoleucine (170mg/100g), Sodium Benzoate and Potassium Sorbate, vitamin C, vitamin E.

| Nutrition facts | Amount/100g (%DRI*) | Amount/serving (41 g) |
|---|---|---|
| Energy kJ (kcal) | 1146 (270) | 470 (111) |
| Protein (g) | 0.3 | 0.1 |
| Carbohydrate (g) | 67.1 | 27.5 |
| Lipid (g) | 0 | 0 |
| Vitamin C (mg) | 22 (37%) | 9 |
| Vitamin E (mg) | 11 (110%) | 4.5 |
| Potassium (mg) | 89 | 36 |
| Sodium (mg) | 90 | 37 |
| L-Leucin (mg) | 170 | 70 |
| L-Valin (mg) | 170 | 70 |
| L-Isoleucin (mg) | 170 | 70 |

| | | |
|---|---|---|
| *%DRI= % of Daily Recommended Intake | | |

### Example 3:

### Comparison of viscosity generating ability of NRC tomato CWM with commercially available tomato fibres

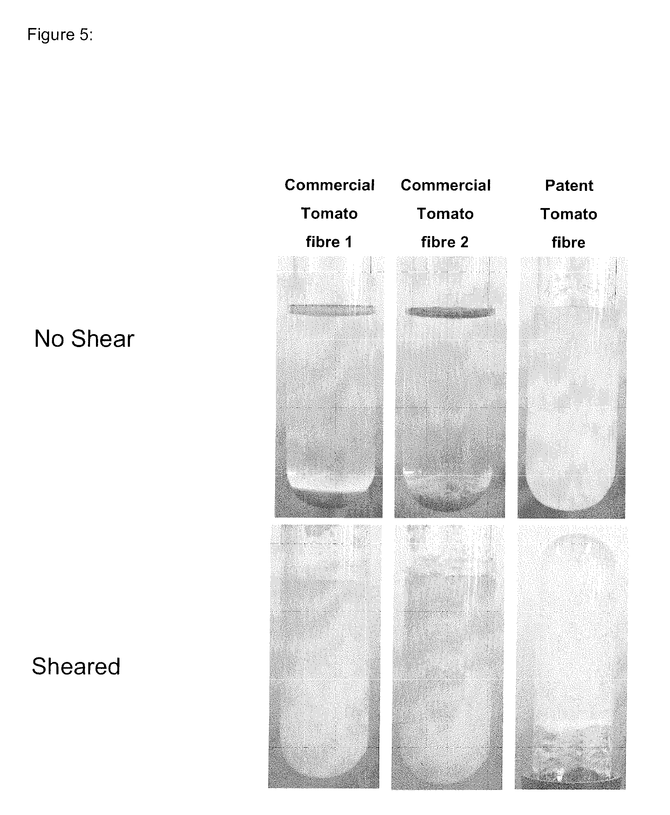

Tomato fibre from 2 different commercial suppliers and the CWM prepared as described above was suspended in water at a concentration of 1%. Without shear the commercial fibres did not disperse and settled out to form a sediment. The CWM prepared by the present inventors on the other hand dispersed readily throughout the volume of the liquid and formed a viscous suspension. Following a high shear treatment (24,000 rpm in an Ultra-Turrax for 60 seconds) the commercial fibres were dispersed throughout the liquid but did not generate the same viscous gel as it is obtained when CWM obtained as described above were used. The results of this comparative study are shown in figure 5.

## Claims

1. Gel comprising a liquid medium and a gel matrix comprising sheared cell wall material from ripe and soft botanical fruits.

2. Gel in accordance with claim 1, **characterized in that** the cell wall material is obtained from the parenchymatous tissue.

3. Gel in accordance with claim 1 or 2, **characterized in that** the cell wall material is chemically unmodified.

4. Gel in accordance with one of claims 1-3, **characterized in that** the cell wall material is sheared, preferably to an average fibre length of about 100 - 0.01 µm, preferably of about 10 - 0. 1 µm, most preferred of about 2 - 0.1 µm.

5. Gel in accordance with one of claims 1-4, **characterized in that** the gel matrix consists of cell wall material from soft botanical fruits.

6. Gel in accordance with one of claims 1-5, **characterized in that** the cell wall material is obtainable by a method comprising the following steps:
- crushing the fruits,
- optionally stopping the enzymatic activity within the fruit material, preferably by a heating step
- separating the cell wall material from the remaining constituents, preferably by the use of a separator or by centrifugation,
- optionally washing to further purify the cell wall material.
- optionally shearing
- optionally washing and/or drying in organic solvents

7. Gel in accordance with one of claims 1-5, **characterized in that** the soft botanical fruits are selected from the group consisting of soft vegetables and soft culinary fruits.

8. Gel in accordance with one of claims 1-7, **characterized in that** the soft botanical fruits are fruit that exhibit an in-vitro swelling of the cell walls during the ripening process, preferably a swelling ratio of ripe fruit vs. unripe fruit of ≥ 2.5.

9. Gel in accordance with one of claims 1-8, **characterized in that** the soft botanical fruits are selected from the group consisting of tomato, kiwi, plums, strawberries, cherries, kaki (persimmon), preferably tomato or kiwi.

10. Gel in accordance with one of claims 1-9, **characterized in that** the liquid medium is a polar medium, preferably comprising water.

11. Gel in accordance with one of claims 1-10, **characterized in that** the liquid medium comprises an emulsion, preferably comprising water and fats, preferably milk fat, long chain fatty acids, medium chain triglycerides, ω3-fatty acids, ω6 fatty acids, plant oils, fish oils or mixtures thereof, in particular preferred is the emulsion milk or a milk-like product.

12. Gel in accordance with one of claims 1-11, **characterized in that** the gel is foamed.

13. Gel in accordance with one of claims 1-12, **characterized in that** it further comprises one or more additives selected from the group consisting of sweeteners, in particular sugars, buffers, colouring agents, preservatives, salts, spices, vitamins, flavouring ingredients, carbohydrates, proteins, bioactives, minerals, acidifying agents, medicaments, or mixtures thereof.

14. Gel in accordance with one of claims 1-13, **characterized in that** the liquid medium represents 60-99.9 weight-% of the gel, preferably 90-99.6 weight-% of the gel, in particular 98-99.5 weight- % of the gel and/or the cell wall material represents 10-0.1 weight-% of the gel, preferably 5.0-0.4 weight-% of the gel, in particular preferred 2-0.5 weight-% of the gel.

15. Gel in accordance with one of claims 1-14, **characterized in that** in consists of edible material.

16. Use of a gel in accordance with claims 1-15 to stabilize a foam.

17. Product comprising a gel in accordance with one of claims 1-15.

18. Product in accordance with claim 17, **characterized in that** it is a cosmetical or a medical product, in particular a moisturizing gel, a shaving cream, a shower gel, a cream or a gel for topical application or a food product, in particular an ambrosia, a jello, a pudding, an ice cream, a confectionery product, a sauce, a functional sports food such as a hydrating gel, gelified water for everyday life, sports gels, gelled water for dried mouth prevention, gelled water with nutrients for elderly, spoonable fruit juice for growing up children, gelling agent in a dessert or a jelly layer in a dessert.

19. Gel building material comprising sheared, preferably chemically unmodified, cell wall material from ripe, soft botanical fruits.

20. Gel building material in accordance with claim 19, **characterized in that** it is obtainable by a method comprising the following steps:
- crushing the fruits,
- optionally stopping the enzymatic activity within the fruit material, preferably by a heating step,
- optionally shearing the cell wall material
- separating the cell wall material from the remaining constituents, preferably by the use of a separator or by centrifugation,
- optionally washing to further purify the cell wall material
- optionally washing and/or drying in organic solvents.

21. Use of a gel building material in accordance with one of claims 19-20 as a binder.

22. Method to prepare a gel comprising the step of adding the gel building material in accordance with one of claims 19-20 to a liquid to be gelled.
